# EUROPEAN PATENT APPLICATION

(11) **EP 1 698 324 A1**
(43) Date of publication of application: **06.09.2006**
(21) Application number: 04256941.8
(22) Date of filing: 09.11.2004
(51) Int. Cl.: A61K 8/41, A61K 8/27, A61K 8/46, A61K 8/36, A61Q 11/00

(54) **Zinc-containing dentifrice compositions having an improved taste**

(71) Applicant: THE PROCTER & GAMBLE COMPANY, Cincinnati, Ohio 45202 (US)
(72) Inventor: Day, Trevor Neil, Egham, Surrey TW20 0JP (GB); Regner, Meinrad, 55127 Mainz (DE)
(74) Representative: Clemo, Nicholas Graham

(57) **Abstract**

The present invention relates to antimicrobial oral compositions comprising a water-soluble zinc salt wherein a surfactant selected from alkali metal or ammonium salts of alkyl sulfoacetate, dialkyl sulfosuccinate, dialkyl sulfosuccinamate; and mixtures thereof; is used in an amount effective to reduce the astringency of the zinc salt.

## Description

### FIELD OF THE INVENTION

The present invention relates to antimicrobial oral compositions, especially dentifrice compositions, comprising a water-soluble zinc salt, wherein a surfactant selected from alkali metal or ammonium salts of alkyl sulfoacetate, dialkyl sulfosuccinate, dialkyl sulfosuccinamate; and mixtures thereof; is used in an amount effective to reduce the astringency of the zinc salt.

### BACKGROUND OF THE INVENTION

The incorporation of zinc compounds, especially in the form of water soluble salts, into oral care products to provide beneficial effects such as anti-plaque and anticalculus, deriving from the antimicrobial properties of the zinc, is well known in the prior art. Also well known is the astringency of such salts, which produces an unpleasant taste in the mouth and is an inhibition to their use in mass appeal products. Complex formation of zinc with anionic counterions such as citrate can reduce its astringency compared to salts such as the chloride but a noticeable taste still generally remains. This imposes some restrictions on the flavours that can successfully be used in a zinc containing oral composition.

Other approaches to reducing the astringency of zinc in oral compositions, especially dentifrice compositions, have been described. These include the approaches described in PCT patent publications WO 94/14407, WO 96/37183, WO 98/37859, WO 99/20238, WO 00/28952, WO 00/61092, WO 03/090702 and the earlier prior art that they recite. Despite the prior research, a need for alternative methods still exists. It has now been found that the inclusion of particular surfactants can reduce the astringency of zinc salts in oral compositions. This improves the taste of the composition and provides greater flexibility in flavour design for such a composition.

### SUMMARY OF THE INVENTION

The present invention relates to an oral composition, especially a dentifrice composition, comprising an antimicrobially effective amount of a water-soluble zinc salt; and an astringency reducing surfactant selected from alkali metal or ammonium salts of alkyl sulfoacetates, dialkyl sulfosuccinates, dialkyl sulfosuccinamates, and mixtures thereof.

### DETAILED DESCRIPTION OF THE INVENTION

Unless specified otherwise, all percentages and ratios herein are by weight of the total composition and all measurements are made at 25°C.

The oral compositions herein can take the form of dentifrice, leave-on oral gels, mouth rinses and chewing gums. Toothpastes, mouth rinses and leave-on oral gels are preferred forms.

The term "dentifrice", as used herein, means a substance for cleaning the teeth which is suitable for application with a toothbrush and is rinsed off after use. It can be a powder, paste, gel, or liquid formulations unless otherwise specified. Dentifrice compositions herein can be single, dual or multi phase preparations. A single phase may comprise a liquid carrier with one or more insoluble particles, such as of a dental abrasive, homogeneously or evenly dispersed within it.

Leave-on oral gels are products which are intended for application to the teeth or gums and, though being intended only for temporary application, as distinct from dental filling materials or permanent dental coatings, are not rinsed off shortly after application, other than by the normal action of saliva. They may be applied locally or spread around the teeth or gums, such as those products described in WO 2004/017933, which are intended for overnight usage. By "mouth rinses" is meant those liquid products which are imbibed or sprayed into the mouth, sluiced around the mouth and then expectorated.

An essential component of the oral compositions herein is a water-soluble zinc salt. Zinc salts are effective as antimicrobial agents. The zinc salt preferably has a solubility of at least 0.1g per 100g water but insoluble materials such as zinc oxide can provide soluble zinc salts if used in conjunction with other solubilizing materials, such as the zinc oxide / sodium citrate combinations described in WO 94/14407. Zinc salts useful in the present invention include zinc chloride, zinc sulfate, zinc nitrate, zinc citrate, zinc gluconate, zinc acetate, zinc lactate and zinc salicylate. Mixed salts such as sodium zinc citrate can also be used. Preferred zinc salts are zinc salts of organic acids such as zinc citrate, zinc lactate, zinc maleate, zinc salicylate, zinc gluconate and zinc ascorbate. More preferred are the zinc salts of carboxylic acids and especially zinc citrate and zinc lactate. Particularly preferred is zinc citrate which is commercially available as a dihydrate. Mixtures of different zinc salts can of course be used.

The zinc salt can be used in any antimicrobially effective amount commensurate with a commercially acceptable product. Excess amounts of zinc will lead to unacceptable taste and may cause problems of compatibility with other ingredients such as e.g., precipitation of anionic polymers. Suitable amounts of zinc salts provide from 0.01 to 1.0% by weight of zinc ions, preferably from 0.05 to 0.3% by weight zinc ions.

The oral compositions herein further include an astringency reducing surfactant selected from alkali metal or ammonium salts of alkyl sulfoacetates, dialkyl sulfosuccinates, dialkyl sulfosuccinamates, and mixtures thereof. Suitable commercially available materials include sodium lauryl sulfoacetate and diethylhexyl sodium sulfoacetate. Alkali metal or ammonium salts of alkyl sulfoacetates are preferred.

The best astringency reducing effects are obtained when near molar equivalents ratios of the astringency reducing surfactant to zinc ion are used. Since commercial surfactants typically comprise mixtures of materials, suitable amounts are best approximated by weight ratio and a suitable weight ratio of the astringency reducing surfactant to zinc ion is from 1:1 to 6:1. Useful absolute amounts of the astringency reducing surfactant are from 0.1 to 2%, preferably from 0.2 to 0.1% by weight of the astringency reducing surfactant, by weight of the oral composition.

The oral compositions herein further comprise an orally acceptable carrier, including customary ingredients such as additional surfactants, fluoride ion sources, anticalculus agents, buffers, abrasive materials, thickening materials, humectants, water, flavours, sweetening agents, colouring agents, and mixtures thereof.

The compositions of the present invention can include surfactants additional to the astringency reducing surfactant. Useful additional surfactant types include anionic, nonionic, cationic and betaine surfactants. Additional anionic surfactants can be included to provide cleaning and foaming properties, and would typically be used in an amount from about 0.1% to about 2.5%, preferably from about 0.3% to about 2.5% and most preferably from about 0.5% to about 2.0% by weight. Cationic surfactants can also be used though care needs to be taken over their compatibility with other ingredients. They would typically be used at levels similar to those of the additional anionic surfactants, as would betaine surfactants. Some nonionic surfactants may be useful at substantially higher levels, such as up to 20% if it is desired to us them to form a ringing gel.

Anionic surfactants useful herein include the water-soluble salts of alkyl sulfates having from 10 to 18 carbon atoms in the alkyl radical and the water-soluble salts of sulfonated monoglycerides of fatty acids having from 10 to 18 carbon atoms. Sodium lauryl sulfate and sodium coconut monoglyceride sulfonates are examples of anionic surfactants of this type. Also useful herein are sarcosinate surfactants, isethionate surfactants and taurate surfactants, such as lauroyl sarcosinate, myristoyl sarcosinate, palmitoyl sarcosinate, stearoyl sarcosinate and oleoyl sarcosinate. All of the foregoing are generally used as their alkali metal or ammonium salts.

Examples of suitable nonionic surfactants include the poloxamers, polyethylene oxide condensates of alkyl phenols, long chain tertiary amine oxides, long chain tertiary phosphine oxides, long chain dialkyl sulfoxides and mixtures of such materials. Preferred betaine surfactants include cocoamidoethyl betaine, cocoamidopropyl betaine, lauramidopropyl betaine and the like.

Other antimicrobial agents may also be employed. Included among such agents are water insoluble non-cationic antimicrobial agents such as halogenated diphenyl ethers, particularly triclosan and essential oils such as thymol. Water soluble antimicrobials include quaternary ammonium salts such as cetyl pyridinium chloride. Enzymes are another type of active that may be used in the present compositions. Useful enzymes include those that belong to the category of proteases, lytic enzymes, plaque matrix inhibitors and oxidases. The oxidases also have whitening/cleaning activity, in addition to anti-microbial properties. Such agents are disclosed in U.S. Patent 2,946,725, Jul. 26, 1960, to Norris et al. and in U.S. Patent 4,051,234, September 27, 1977 to Gieske et al.

Another preferred optional agent is an anticalculus agent, such as a soluble polyphosphate, polyphosphonate or pyrophosphate. The pyrophosphates used in the present compositions can be any of the alkali metal pyrophosphate salts. Specific salts include tetra alkali metal pyrophosphate, dialkali metal diacid pyrophosphate, trialkali metal monoacid pyrophosphate and mixtures thereof, wherein the alkali metals are preferably sodium or potassium. The salts are useful in both their hydrated and unhydrated forms. An effective amount of pyrophosphate salt useful in the present composition is generally enough to provide at least 1.0% pyrophosphate ion, preferably from about 1.5% to about 6%, more preferably from about 3.5% to about 6% of such ions. It is to be appreciated that the level of pyrophosphate ions is that capable of being provided to the composition (i.e., the theoretical amount at an appropriate pH) and that pyrophosphate forms other than P2O7-4 (e.g., (HP2O7-3)) may be present when a final product pH is established. The pyrophosphate salts are described in more detail in Kirk & Othmer, Encyclopedia of Chemical Technology, Second Edition, Volume 15, Interscience Publishers (1968). Also useful are the soluble polyphosphates such as sodium tripolyphosphate and sodium hexametaphosphate. Other long chain anticalculus agents of this type are described in WO 98/22079. Particularly preferred for use herein are sodium polyphosphate salts containing about 15 to about 25 phosphate units.

Another preferred ingredient is a water-soluble fluoride compound, used in an amount sufficient to give a fluoride ion concentration in the composition of from about 0.0025% to about 0.5% by weight, to provide anticaries effectiveness. A wide variety of fluoride ion-yielding materials can be employed as sources of soluble fluoride in the present compositions. Representative fluoride ion sources include stannous fluoride, sodium fluoride, potassium fluoride, sodium monofluorophosphate and many others. Stannous fluoride and sodium fluoride are particularly preferred, as well as mixtures thereof. If, however, sodium fluoride is used in combination with the long chain polyphosphates then it is preferably kept in a separate phase.

In preparing toothpaste or gels, it is often necessary to add a thickener or binder to provide a desirable consistency of the composition, to provide desirable active release characteristics upon use, to provide shelf stability, and to provide stability of the composition, etc. Thickening agents can include carboxyvinyl polymers, carrageenan, nonionic cellulose derivatives such as hydroxyethyl cellulose, and water soluble salts of cellulose derivatives such as sodium carboxymethylcellulose. It should be recognised though that the anionic polymers such as carboxyvinyl polymers can interact with the zinc salts in a way which reduces the effectiveness of the zinc and the interaction may also have an undesirable effect on the rheology of the composition. Natural gums such as gum karaya, xanthan gum, gum arabic, and gum tragacanth can also be used herein. A highly preferred thickener system comprises a mixture of xanthan gum and hydroxyethyl cellulose, which provides a thickened composition without stringiness.

Another optional but preferred component of the compositions herein is a humectant. The humectant serves to keep the dentifrice from hardening upon exposure to air, to give a moist feel to the mouth, and, for particular humectants, to impart a desirable sweetness of flavour. The humectant, on a pure humectant basis, generally comprises from about 5% to about 70%, preferably from about 15% to about 45%, by weight of the composition. Suitable humectants include edible polyhydric alcohols such as glycerin, sorbitol, xylitol, butylene glycol, polyethylene glycol, and propylene glycol, especially sorbitol and glycerin.

A preferred ingredient for a dentifrice herein is a dental abrasive. Abrasives serve to polish the teeth and /or remove surface deposits. The abrasive material contemplated for use herein can be any material which does not excessively abrade dentine. Suitable abrasives include insoluble phosphate polishing agents, include various calcium phosphates such as, for example, dicalcium phosphate, tricalcium phosphate, calcium pyrophosphate, beta-phase calcium pyrophosphate, dicalcium phosphate dihydrate, anhydrous calcium phosphate, insoluble sodium metaphosphate, and the like. Also suitable are chalk-type abrasives such as calcium and magnesium carbonates, silicas including xerogels, hydrogels, aerogels and precipitates, alumina and hydrates thereof such as alpha alumina trihydrate, aluminosilicates such as calcined aluminium silicate and aluminium silicate, magnesium and zirconium silicates such as magnesium trisilicate and thermosetting polymerised resins such as particulate condensation products of urea and formaldehyde, polymethylmethacrylate, powdered polyethylene and others such as disclosed in US-A-3,070,510, December 25, 1962. Mixtures of abrasives can also be used. The abrasive polishing materials generally have an average particle size of from about 0.1 to about 30 microns, preferably from about 5 to 15 microns.

Silica dental abrasives of various types offer exceptional dental cleaning and polishing performance without unduly abrading tooth enamel or dentin. The silica abrasive can be precipitated silica or silica gels. Suitable precipitated silica materials include those marketed by the J. M. Huber Corporation under the tradename, "Zeodent®", particularly the silicas carrying the designation Zeodent® 119 or Zeodent® 118. These silica abrasives are described in US-A-4,340,583, July 29, 1982 and WO 96/09809.

Suitable abrasive levels for a dentifrice are from 1% to 40%, preferably at least 2%, such as from 2% to 20%, more preferably at least 5%, such as from 5% to 25%.

Flavouring and sweetening agents are preferably also included in the present compositions. It is an advantage of the present invention that a wide range of flavouring ingredients can be used. Suitable flavouring agents and sweetening agents are well known in the art. Suitable flavour levels in the present oral compositions herein are from 0.1% to 5.0%, more preferably from 0.5% to 2.0%, and most preferably from about 0.7% to about 1.8%, by weight. Typically, a flavour oil will be manufactured in a separate step and will comprise multiple components, natural and/or synthetic in origin, in order to provide a balanced flavour which is acceptable to a broad range of people. Flavour components can be selected from mint, spice, fruit, citrus, herbal, medicinal, and common food flavour types (e.g. chocolate). Illustrative, but non-limiting examples of such components include hydrocarbons such as limonene, caryophyllene, myrcene, and humulene; alcohols such as menthol, linalool, 3-decanol, and pinocarveol; ketones such as piperitone, menthone, spicatone, and l-carvone; aldehydes such as acetaldehyde, 3-hexanal, or n-octanal; oxides such as menthofuran, piperitone oxide, or carvyl acetate-7,7 oxide; acids such as acetic and ocenoic; and sulphides such as dimethyl sulphide. Components also include esters such as menthyl acetate, benzyl isobutyrate, and 3-octyl acetate. The flavour components may also include essential oils such as peppermint oils from e.g., Mentha piperita and Mentha arvensis; spearmint oils such as those from Mentha cardiaca and Mentha spicata; sage oil, parsley oil, marjoram oil, cassia oil, clove bud oil, cinnamon oil, orange oil, eucalyptus oil and anise oil. Other suitable components are cinnamic aldehyde, eugenol, ionone, anethole, eucalyptol, thymol, methyl salicylate, vanillin, ethyl vanillin, and vanilla extracts. Whilst it is an advantage of the present invention that it provides for greater flexibility in flavour selection, those flavouring systems described in the art as being particularly suitable for zinc formulae, such as those described in US-A-6,306,372 and WO 00/28952, can of course be used. Flavour components are described in more detail in Fenaroli's Handbook of Flavor Ingredients, Third Edition, Volumes 1 & 2, CRC Press, Inc. (1995), and Steffen Arctander's Perfume and Flavour Chemicals, Volumes 1 & 2, (1969). A physiological cooling agent can also be incorporated into the flavour oil. The coolant can be any of a wide variety of materials. Included among such materials are carboxamides, menthol, acetals, ketals, diols, and mixtures thereof. Preferred coolants in the present compositions are the p-menthane carboxamide agents such as N-ethyl-p-menthane-3-carboxamide, (known commercially as "WS-3") and mixtures thereof and menthone glycerine acetal (known commercially as "MGA"). Further disclosure of coolants suitable for the present invention are discussed in WO97/06695.

The compositions may further include usual pigments and colorants, such as titanium dioxide.

Water employed in the oral compositions herein should preferably be of low ion content and free of organic impurities. Water generally comprises from about 10% to about 50%, and preferably from about 20% to about 40%, by weight of a toothpaste herein. These amounts of water include the free water which is added plus that which is introduced with other materials, such as with sorbitol. Higher amounts of water will generally be used for mouthrinses which may further comprise other solvents such as ethanol and propylene glycol.

The pH of the present compositions which should generally be less than 8, can be adjusted, through the use of buffering agents to a preferred range of 5 to 7, more preferably from about 6.0 to about 6.7. When the zinc salt is a carboxylic acid buffering is generally provided by this salt. If additional buffering is required or buffering at a different pH is required, additional suitable buffering agents that may be employed include sodium acid pyrophosphate, citric acid, sodium citrate, tartaric acid, sodium tartrate, acetic acid and sodium acetate. The pH of the dentifrice is measured from a 3:1 aqueous slurry of dentifrice.

### Examples

In each of the following examples, mixing in the main vessel is carried out under a partial vacuum (-0.09 MPa) to avoid air entrainment and mixing at each stage is continued until the mixture is homogeneous.

### Example 1

There follow three examples of toothpaste compositions according to the invention.

| Formula: | | A | B | C |
|---|---|---|---|---|
| Ingredient | | % | % | % |
| Sorbitol (70% aq.)* | | 31.442 | 28.899 | 31.410 |
| Hydrated silica amorphous (Zeodent® 119) | | 20.000 | 23.000 | 23.000 |
| Purified water | | 21.381 | 13.713 | 13.713 |
| Glycerin | | 8.000 | 8.000 | 8.000 |
| PEG-6 | | 6.000 | 6.000 | 6.000 |
| Tetrapotassium pyrophosphate (60% aq.)* | | - | 3.159 | - |
| Tetrasodium pyrophosphate | | - | 1.908 | - |
| Disodium pyrophosphate | | - | 1.344 | - |
| Sodium tripolyphosphate | | - | - | 4.000 |
| Sodium lauryl sulfate (28% aq.)* | | 6.000 | 7.000 | 7.000 |
| Sodium lauryl sulfoacetate (10% aq.)** | | 2.800 | 2.800 | 2.800 |
| Zinc citrate dihydrate | | 0.531 | 0.531 | 0.531 |
| Titanium dioxide | | 0.525 | 0.525 | 0.525 |
| Xanthan gum | | 1.000 | 0.600 | 0.800 |
| Hydroxyethyl cellulose | | 0.500 | 0.700 | 0.400 |
| Sodium fluoride | | 0.321 | 0.321 | 0.321 |
| Sodium saccharin | | 0.300 | 0.300 | 0.300 |
| Flavour | | 1.200 | 1.200 | 1.200 |
| | Total: | 100.000 | 100.000 | 100.000 |

| | | | | |
|---|---|---|---|---|
| * As solution | | | | |
| ** Pre-mix in purified water | | | | |

### Making Instructions

Add the water, sorbitol, zinc citrate dihydrate, sodium fluoride, sodium saccharin and, where relevant, the phosphates, to the main mixing vessel and mix. Disperse the xanthan gum and hydroxyethyl cellulose in glycerin, add the mixture to the main vessel and mix. Pre-mix the silica and titanium dioxide, add to the main vessel and mix. Add the PEG-6, sodium lauryl sulfate solution, sodium lauryl sulfoacetate solution and flavour to the main vessel and mix.

### Example 2

Example 2 is a further toothpaste according to the invention.

| Ingredient | | (% w/w) |
|---|---|---|
| Sorbitol (70% aq.)* | | 30.242 |
| Hydrated silica amorphous (Zeodent® 119) | | 20.000 |
| Purified water | | 21.381 |
| Glycerin | | 8.000 |
| PEG-6 | | 6.000 |
| Sodium lauryl sulfate (28% aq.)* | | 6.000 |
| Diethylhexyl sodium sulfoacetate (10% aq)** | | 4.000 |
| Zinc citrate dihydrate | | 0.531 |
| Titanium dioxide | | 0.525 |
| Xanthan gum | | 1.000 |
| Hydroxyethyl cellulose | | 0.500 |
| Sodium fluoride | | 0.321 |
| Sodium saccharin | | 0.300 |
| Flavour | | 1.200 |
| | Total: | 100.000 |

| | | |
|---|---|---|
| * As solution | | |
| ** Pre-mix in purified water | | |

### Making Instructions

Add the water, sorbitol, zinc citrate dihydrate, sodium fluoride and sodium saccharin to the main mixing vessel and mix. Disperse the xanthan gum and hydroxyethyl cellulose in glycerin, add the mixture to the main vessel and mix. Pre-mix the silica and titanium dioxide, add to the main vessel and mix. Add the PEG-6, sodium lauryl sulfate solution, diethylhexyl sodium sulfoacetate solution and flavour to the main vessel and mix.

### Example 3

This is a leave-on overnight gel according to the invention.

| Ingredient | | (% w/w) |
|---|---|---|
| Purified water | | 70.700 |
| Glycerin | | 15.000 |
| PEG-6 | | 6.000 |
| Sodium lauryl sulfoacetate (10% aq.)** | | 4.000 |
| Zinc citrate dihydrate | | 1.000 |
| Xanthan gum | | 1.000 |
| Hydroxyethyl cellulose | | 0.500 |
| Sodium saccharin | | 0.300 |
| Flavour | | 1.500 |
| | Total: | 100.000 |

| | | |
|---|---|---|
| ** Pre-mix in purified water | | |

### Making Instructions:

Add the water, zinc citrate dihydrate, and sodium saccharin to the main mixing vessel and mix. Disperse the xanthan gum and hydroxyethyl cellulose in glycerin, add the mixture to the main vessel and mix. Pre-mix the silica and titanium dioxide, add to the main vessel and mix. Add the PEG-6, sodium lauryl sulfoacetate solution and flavour to the main vessel and mix.

## Claims

1. An oral composition comprising:
a) an antimicrobially effective amount of a water-soluble zinc salt;
b) an astringency reducing surfactant selected from alkali metal or ammonium salts of alkyl sulfoacetates, dialkyl sulfosuccinates, dialkyl sulfosuccinamates, and mixtures thereof; and
c) an orally acceptable carrier.

2. An oral composition according to Claim 1 wherein the zinc salt is selected from zinc salts of organic acids, preferably from zinc salts of carboxylic acids.

3. An oral composition according to Claim 2 wherein the zinc salt is selected from zinc citrate, zinc lactate and mixtures thereof, preferably wherein the zinc salt comprises zinc citrate.

4. An oral composition according to any preceding claim comprising from 0.01 to 1.0% by weight of zinc ions, preferably from 0.05 to 0.3% by weight zinc ions.

5. An oral composition according to any preceding claim wherein the weight ratio of the astringency reducing surfactant to zinc ion is from 1:1 to 6:1.

6. An oral composition according to any preceding claim wherein the astringency reducing surfactant is selected from alkali metal or ammonium salts of an alkyl sulfoacetate; and mixtures thereof.

7. An oral composition according to any preceding claim comprising from 0.1 to 2%, preferably from 0.2 to 1% by weight of the astringency reducing surfactant.

8. An oral composition according to any preceding claim further comprising sodium alkyl sulfate.

9. An oral composition according to any preceding claim wherein the composition has a pH of less than 8, preferably from 5 to 7, more preferably from 6.0 to 6.7.

10. An oral composition according to any preceding claim wherein the composition comprises a thickener system comprising xanthan gum and hydroxyethyl cellulose.
